# EUROPÄISCHE PATENTSCHRIFT

(11) **EP 1 827 532 B1**
(45) Veröffentlichungstag und Bekanntmachung des Hinweises auf die Patenterteilung: **27.03.2019**
(21) Anmeldenummer: 05807459.2
(22) Anmeldetag: 11.11.2005
(51) Int. Cl.: A61M 1/34

(54) **VORRICHTUNG ZUR ABREICHERUNG WENIGSTENS EINER KOMPONENTE EINES FLUIDEN MEDIUMS**
DEVICE FOR DOWNGRADING AT LEAST ONE COMPONENT OF A FLUID MEDIUM
DISPOSITIF POUR EPUISER AU MOINS UN COMPOSANT D'UN MILIEU FLUIDE

(30) Priorität: 12.11.2004 DE 102004054747
(43) Veröffentlichungstag der Anmeldung: 05.09.2007
(73) Patentinhaber: Fresenius Medical Care Deutschland GmbH, 61352 Bad Homburg (DE)
(72) Erfinder: WÜPPER, Andreas, 64572 Büttelborn (DE)
(74) Vertreter: Herrmann, Uwe
(86) Internationale Anmeldenummer: PCT/EP2005/012110
(87) Internationale Veröffentlichungsnummer: WO 2006/050970

(56) Entgegenhaltungen:
- EP-A- 0 693 298
- EP-A- 1 348 458
- WO-A-98/19592
- WO-A-2004/091694
- US-A- 4 976 682
- US-A1- 2004 182 787

## Beschreibung

Die vorliegende Erfindung betrifft eine Vorrichtung zur Abreicherung wenigstens einer Komponente eines fluiden Mediums gemäß dem Oberbegriff des Anspruchs 1. Eine solche Vorrichtung ist aus der US 4,976,682 bekannt. Diese Druckschrift bezieht sich auf eine Vorrichtung, mittels welcher aus Wunden austretendes Blut gesammelt, gereinigt und wieder in dem Blutkreislauf eines Patienten geführt wird.

Häufig besteht die Aufgabe, aus Fluiden, beispielsweise aus flüssigen Gemischen einzelne Komponenten abzutrennen, wobei dazu sehr unterschiedliche Verfahren herangezogen werden können. Ein Verfahren zur Abreicherung von Wert- oder Schadstoffen ist beispielsweise die Adsorption, bei der einer oder mehrere Stoffe eines Gemisches an einen Adsorber gebunden und auf diese Weise von den weiteren Gemischkomponenten abgetrennt werden. Neben der Adsorption sind auch zahlreiche andere Trennverfahren, wie beispielsweise Membrantrennverfahren bekannt.

Die vorgenannten Trennverfahren haben häufig den Nachteil, daß deren Effizienz nicht zufriedenstellend ist bzw. im Laufe des Trennvorgangs abnimmt. Beispielsweise besteht bei der Verwendung von Adsorbern das Problem, daß diese bei geringer Beladung des fluiden Mediums mit der abzutrennenden Komponente nur eine verhältnismäßig geringe Menge dieser Komponente adsorbieren, so daß die Effizienz dieses Trennverfahrens in diesem Fall entsprechend gering ist. Entsprechendes gilt beispielsweise für Membranverfahren, deren Effizienz bei kleinen Konzentrationen ebenfalls verhältnismäßig gering ist.

Es ist die Aufgabe der vorliegenden Erfindung eine Vorrichtung zur Abreicherung wenigstens einer Komponente eines fluiden Mediums dahingehend weiterzubilden, daß die Effizienz der Abreicherung dieser Komponente erhöht wird.

Diese Aufgabe wird durch eine Vorrichtung mit den Merkmalen des Anspruchs 1 gelöst. Danach ist vorgesehen, daß die Konzentration der wenigstens einen abzureichenden Komponente vor deren Abreicherung erhöht wird. Die erfindungsgemäße Vorrichtung beruht somit darauf, den abzureichernden Stoff vor seiner Abreicherung aufzukonzentrieren. Dem Mittel zur Abreicherung der Komponente, nämlich dem Adsorber wird somit eine höhere Konzentration geboten, so daß die Abtrennung der betreffenden Komponente entsprechend effektiver erfolgt. Dies ist im Falle der Verwendung von Adsorbern darauf zurückzuführen, daß entsprechend der Adsorptionsisotherme eine höhere Konzentration der betreffenden Komponente in dem fluiden Medium zu einer entsprechend höheren Beladung des Adsorbers mit dieser Komponente führt. Ein Verfahren zur Abreicherung wenigstens einer Komponente eines fluiden Mediums kann entsprechend dieser und der nachstehenden Merkmale ausgeführt werden.

Bei dem fluiden Medium kann es sich um eine Flüssigkeit handeln. Beispielsweise handelt es sich um Blut oder Blutplasma.

Die Erhöhung der Konzentration erfolgt durch Ultrafiltration.

Erfindungsgemäß ist vorgesehen, dass dem fluiden Medium nach Abreicherung der Komponente ein Substitutionsmedium zugeführt wird. Vorteil der Zuführung des Substitutionsmediums ist vor allem die Volumenerhaltung. So ist erreichbar, daß die Konzentration von Komponenten, die nicht abgereichert werden durch das Verfahren insgesamt nicht oder kaum verändert wird. Dementsprechend besteht eine Weiterbildung der Erfindung darin, daß das fluide Medium wenigstens eine Komponente enthält, die bei der Abreicherung der abzureichenden Komponente nicht abgereichert wird, und daß das Substitutionsmedium in einer Menge zugeführt wird, daß die Konzentration der nicht abgereicherten Komponente des Substitutionsmediums in etwa der Konzentration dieser Komponente vor der Konzentrationserhöhung entspricht.

Denkbar ist jedoch auch, das die Konzentration der nicht abgereicherten Komponente nach Zugabe des Substitutionsmediums nicht der Konzentration dieser Komponente vor der Konzentrationserhöhung entspricht. Ob die Konzentration der nicht abzureichernden Komponenten unverändert bleibt oder sich insgesamt ändert, ist substanzabhängig.

Grundsätzlich ist es möglich, daß insgesamt nur die Konzentration der abzureichernden Komponente verringert wird, wohingegen die Konzentrationen der weiteren Komponenten insgesamt keine Änderung erfahren.

Die Abreicherung der abzureichernden Komponente erfolgt mittels der Adsorption.

Erfindunggemäß ist vorgesehen, daß die Vorrichtung einen extrakorporalen Kreislauf aufweist. Dabei kann in dem extrakorporalen Kreislauf ein Plasmafilter vorgesehen sein, wobei es sich bei dem fluiden Medium um in dem Plasmafilter vom Blut abgetrenntes Blutplasma oder fraktioniertes Plasma handeln kann. Es kann somit auch ein Filter zum Einsatz kommen, der fraktioniertes Plasma durchlässt (z.B. "Albuflow", lässt Albumin durch, hält jedoch Immunglobuline zurück). Auf diese Weise läßt sich mit der erfindungsgemäßen Vorrichtung eine Adsorptionstherapie des Plasmas durchführen.

In weiterer Ausgestaltung der Erfindung ist vorgesehen, daß die Konzentrationserhöhung der abzureichernden Komponente mittels Ultrafiltration durchgeführt wird und daß der Ultrafilter zur Dialyse genutzt wird. Ist eine Eliminierung von kleinmolekularen Stoffen durch Dialyse erforderlich, wie dies beispielsweise bei der Leberunterstützungstherapie der Fall ist, kann der Ultrafilter dementsprechend auch optional zur Dialyse mitgenutzt werden.

In weiterer Ausgestaltung der Erfindung ist vorgesehen, daß es sich bei dem fluiden Medium um Blut oder Blutplasma handelt und daß die wenigstens eine abzureichernde Komponente eine albumingebundene Substanz, Immunglobuline, Interleukin oder LDL-Cholesterin (Low-Density-Lipoprotein-Cholesterin) ist.

Die Konzentrationserhöhungseinheit ist als Ultrafilter ausgeführt. Erfindungsgemäß vorgesehen ist der Einsatz eines Adsorbers als Abreicherungseinheit.

Wie oben ausgeführt, ist vorgesehen, daß die Vorrichtung ferner einen extrakorporalen Kreislauf zur Führung von Blut oder einem oder mehreren Blutbestandteilen umfaßt, wobei in dem extrakorporalen Kreislauf die Konzentrationserhöhungseinheit sowie die Abreicherungseinheit angeordnet sind.

Von der Konzentrationserhöhungseinheit kann eine Leitung abzweigen, die stromabwärts der Abreicherungseinheit in den extrakorporalen Kreislauf mündet. Diese Leitung dient zur Zuführung des bei der Konzentrationserhöhung abgetrennten Mediums, beispielsweise des Filtrats, zu dem Blut bzw. Blutplasma nach der Abreicherung der abzureichernden Komponente.

Erfindungsgemäß ist vorgesehen, daß stromabwärts der Abreicherungseinheit eine Leitung in den extrakorporalen Kreislauf mündet, die mit einer Quelle eines Substitutionsmediums in Fluidverbindung steht. Somit ist es möglich, das bei der Konzentrationserhöhung abgetrennte Medium nicht zum Blut/Blutplasma zurückzuführen, sondern durch eine Substitutionsflüssigkeit zu ersetzen.

Auch eine Kombination beider vorgenannter Vorgehensweisen ist vorstellbar.

In bevorzugter Ausgestaltung der Erfindung weist der extrakorporale Kreislauf einen Erst- und einen Zweitkreislauf auf, wobei der Erstkreislauf mit einem Patienten in Fluidverbindung steht oder verbindbar ist und wobei der Zweitkreislauf mit dem Erstkreislauf mittels eines Plasmafilters in Verbindung steht und die Konzentrationserhöhungseinheit sowie die Abreichungseinheit umfaßt. Eine derartige Anordnung dient dazu, das mittels des Plasmafilters aus dem Blut gewonnene Plasma einer Adsorptionstherapie zu unterwerfen, wobei vor der Adsorption erfindungsgemäß eine Konzentrationserhöhung durch Ultrafiltration stattfindet.

Des Weiteren kann vorgesehen sein, daß an die Konzentrationserhöhungseinheit ein Dialysatskreislauf angeschlossen ist oder anschließbar ist. Handelt es sich dabei um einen Ultrafilter, kann dieser zusätzlich zur Dialyse verwendet werden. In diesem Fall wird der Ultrafilter auf der einen Seite der Membran von Blut bzw. Blutplasma und auf der anderen Seite der Membran von Dialysat durchströmt.

Weitere Einzelheiten und Vorteile der Erfindung werden anhand eines in der Zeichnung dargestellten Ausführungsbeispiels näher erläutert. Es zeigen:
- Figur 1:: Adsorptionsisotherme: adsorbierte Menge der Komponente als Funktion der Konzentration dieser Komponente in der Flüssigkeit,
- Fig. 2a, 2b:: Verfahrensschema der "Ultra-Absorption" mit Rückführung des Ultrafiltrats (nicht Bestandteil der vorliegenden Erfindung),
- Figur 3:: Verfahrensschema der "Ultra-Adsorption" mit Zuführung frischer Substitutionslösung,
- Figur 4:: Erläuterung des Effektes der ultrafiltrationsverstärkten Adsorption anhand der Adsorptionsisotherme,
- Figur 5:: Schematische Darstellung des Versuchsaufbaus zur Ermittlung des Effektes der Konzentrationserhöhung einer Komponente vor deren Abreicherung,
- Figur 6.: Darstellung der Bilirubinkonzentration in Abhängigkeit von der Zeit bei unterschiedlichen Ultrafiltrationsraten und
- Figur 7:: Darstellung der Bilirubin-Clearance in Abhängigkeit von der Bilirubinkonzentration bei unterschiedlichen Ultrafiltrationsraten.

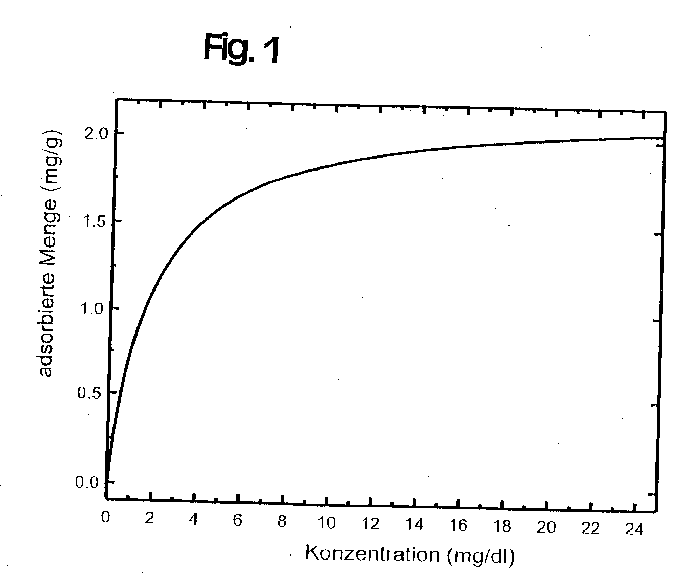

Figur 1 zeigt die sogenannte Adsorptionsisotherme, die die auf einem Adsorber adsorbierte Menge in Abhängigkeit von der Konzentration der betreffenden Komponente in der Lösung wiedergibt.

Adsorber dienen beispielsweise zur extrakorporalen Blutreinigung und dabei zur Eliminierung von Substanzen, die durch Dialyse und Hemofiltration nicht entfernbar sind. Dialyse und Hemofiltration entfernen Substanzen, die wasserlöslich sind und ein Molekulargewicht besitzen, daß unterhalb der Ausschlußgrenze des Dialysators bzw. Hemofilters liegt. Diese Ausschlussgrenze(< 60 kD) ist so gewählt, daß Albumin, das Hauptprotein des Blutes, zurückgehalten wird.

Wie oben ausgeführt, dienen Adsorptionsverfahren zur Blutreinigung von Stoffen, die albumingebunden sind oder aufgrund ihres hohen Molekulargewichtes nicht oder nur unzureichend durch Dialyse oder Hemofiltration entfernbar sind. Substanzen, die aufgrund ihres hohen Molekulargewichtes durch Adsorption anstatt durch Dialyse entfernt werden, sind beispielsweise LDL (Behandlung von Hypercholesterinämie) und Immunglobuline (Behandlung von Autoimmunerkrankungen, Transplantationsvorsorge). Bei den albumingebundenen Substanzen handelt es sich beispielsweise um das wasserunlösliche Bilirubin, das im Blut an Albumin gebunden transportiert wird. Es ist nur durch absorptive Verfahren entfernbar, die beispielsweise bei der Leberunterstützungstherapie zum Einsatz kommen.

Wie aus Figur 1 ersichtlich, ist die Substanzmenge, die von einem Adsorber gebunden werden kann von der Konzentration der zu adsorbierenden Substanz in der Lösung, beispielsweise im Blut oder im Plasma, abhängig. Aus der Adsorptionsisotherme gemäß Figur 1 ergibt sich, daß bei höheren Konzentrationen auch größere Beladungen vorliegen, d. h. größere Mengen an den Adsorber gebunden werden können. Eine Konzentrationserhöhung führt insbesondere dann zu einer wesentlichen Änderung in der adsorbierten Menge, wenn die Konzentration im stark ansteigenden Teil der Kurve liegt.

Im Laufe einer Behandlung mit Adsorbern kann beobachtet werden, daß die Entfernungsleistung, d. h. die Clearance, abnimmt. Ein Grund dafür ist die zunehmende Beladung des Adsorbers. Sehr häufig hat die Clearance aber schon stark abgenommen, noch ehe die maximale Beladung des Adsorbers erreicht wird. Dies ist darauf zurückzuführen, daß die Konzentration in der zu reinigenden Substanz in dem fluiden Medium, beispielsweise im Blut oder Plasma in den Bereich der starken Steigung der Adsorptionsisotherme gefallen ist, so daß die adsorbierte Menge dementsprechend ebenfalls gering ist.

Die erfindungsgemäße Vorrichtung basiert darauf, den abzureichernden Stoff bzw. den zu absorbierenden Stoff in dem Fluid, beispielsweise im Blut oder Plasma, vor der Abreicherung aufzukonzentrieren. Gemäß der Erfindung findet die Abreicherung durch Adsorption und die Aufkonzentrierung durch Ultrafiltration statt. Auf diese Weise kann erreicht werden, daß dem Adsorber eine höhere Konzentration geboten wird, so daß die Adsorption gemäß Figur 1 effektiver erfolgt, da die Aufkonzentrierung zu einer höheren Beladung des Adsorbers führt.

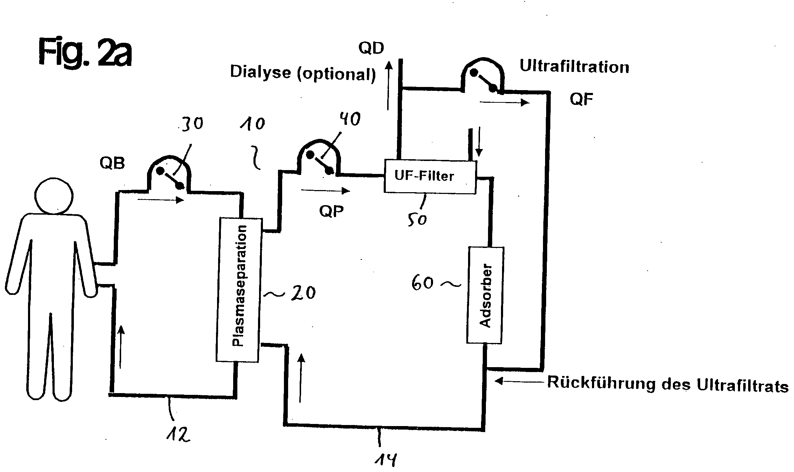

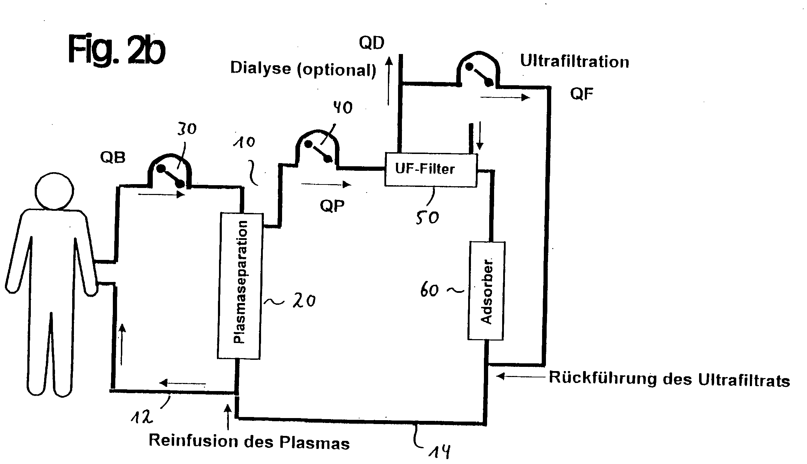

Figur 2a zeigt ein Verfahrensschema der Kombination aus Ultrafiltration und Adsorption ("Ultra-Adsorption"). Wie aus Figur 2 ersichtlich, besteht der extrakorporale Kreislauf 10 aus einem Erstkreislauf 12 und einem Zweitkreislauf 14. Beide Kreisläufe 12, 14 sind durch den Plasmafilter 20 miteinander verbunden. In dem Plasmafilter 20 wird aus dem in dem Erstkreislauf 12 strömenden Blut Blutplasma entnommen. Das Blutplasma zirkuliert durch den Zweitkreislauf 14.

Die Zirkulation des Blutes im Erstkreislauf 12 erfolgt mittels der Blutpumpe 30. Die Zirkulation des Blutplasmas im Zweitkreislauf 14 erfolgt mittels der Plasmapumpe 40. Wie aus Figur 2a ersichtlich, befindet sich in dem Zweitkreislauf 14 der Ultrafilter 50. Diesem ist stromabwärts nachgeschaltet der Adsorber 60. In dem Ultrafilter 50 erfolgt eine Aufkonzentrierung des Plasmas und somit auch eine Konzentrationserhöhung der abzureichernden Komponente, wie beispielsweise Bilirubin. Somit wird der Adsorber 60 mit einer aufkonzentrierten Lösung beaufschlagt, was zur Folge hat, daß die Abtrennung der abzureichernden Komponente aufgrund der höheren adsorbierten Menge entsprechend der Adsorptionsisotherme gemäß Figur 1 effektiver erfolgt.

Wie aus Figur 2a weiter ersichtlich, wird das bei der Ultrafiltration in dem Ultrafilter 50 gewonnene Ultrafiltrat mittels einer Pumpe und einer Ultrafiltrationsleitung stromabwärts des Adsorbers 60 wieder dem Zweitkreislauf 14 zugeführt. Dadurch wird eine Volumenerhaltung erreicht. Ferner lässt sich auf diese Weise erreichen, daß die Konzentration zumindest einiger der nicht in dem Adsorber 60 abgereicherten Komponenten insgesamt nicht geändert wird., d. h. der Konzentrationserhöhung im Ultrafilter 50 folgt eine entsprechende Verdünnung. Ob insgesamt eine Konzentrationsänderung eintritt, ist im wesentlichen substanzabhängig.

Alternativ zu dem in Fig. 2a dargestellten System ist es möglich, das Plasma in dem Zweitkreislauf 14 direkt in den Erstkreislauf 12 zu reinfundieren, wie dies in Fig. 2b dargestellt ist.

Fig. 2b zeigt eine Variante des in Figur 2a dargestellten Verfahrensschemas ("Reinfusion"). Dabei sind Erstkreislauf 12 und Zweitkreislauf 14 nur stromauf durch den Plasmafilter 20 getrennt. Stromab erfolgt eine direkte Infusion des Blutplasmas des Zweitkreislaufs 14 in das Vollblut des Erstkreislaufs 12. Diese Variante hat gegenüber der in Figur 2a dargestellten Variante den Vorteil, dass die Menge des vom Erstkreislauf 12 in den Zweitkreislauf 14 übertretenden Plasmas durch die Plasmapumpe 40 gesteuert werden kann. Bei dem in Figur 2a dargestellten Verfahren ("Rezirkulation") erfolgt der Übertritt von beladenem Plasma aus dem Erstkreislauf 12 in den Zweitkreislauf 14 bzw. von abgereichertem Plasma aus dem Zweitkreislauf 14 in den Erstkreislauf 12 rein passiv und im wesentlichen unabhängig von der Förderrate der Plasmapumpe 40.

Bei der Verfahrensvariante "Reinfusion" gemäß Fig. 2b bedarf es weiterer Sicherheitsmaßnahmen, um bei einem Erstfehler "undichte Adsorberkartusche" einen Übertritt von Adsorbermaterial in den systematischen Blutkreislauf des Patienten zu verhindern. Diese Sicherheitsmaßnahmen sind hier nicht gesondert dargestellt.

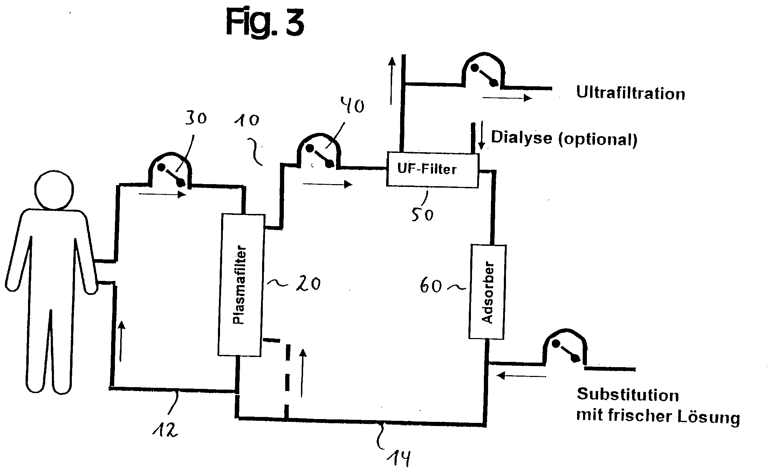

Alternativ zu der in Fig. 2a, 2b dargestellten Anordnung ist es denkbar, gemäß Figur 3 das Ultrafiltrat zu verwerfen und stromabwärts des Adsorbers frische Substitutionslösung zuzugeben, wie dies in Figur 3 dargestellt ist.

Das entnommene Ultrafiltrat kann dem Blut bzw. Plasma somit entweder wieder zugeführt werden oder durch eine Substitutionslösung ersetzt werden. Auch eine Kombination beider Möglichkeiten ist denkbar, wobei nur Ausführungsformen bei denen eine Substitutionslösung stromabwärts der Abreicherungseinheit zugegeben wird unter die Erfindung fallen.

Fig. 3 zeigt die beiden Varianten "Rezirkulation und Reinfusion" als alternative Flußwege. Insofern nehmen wir Bezug zu den obigen Erläuterungen zu Fig. 2a, 2b.

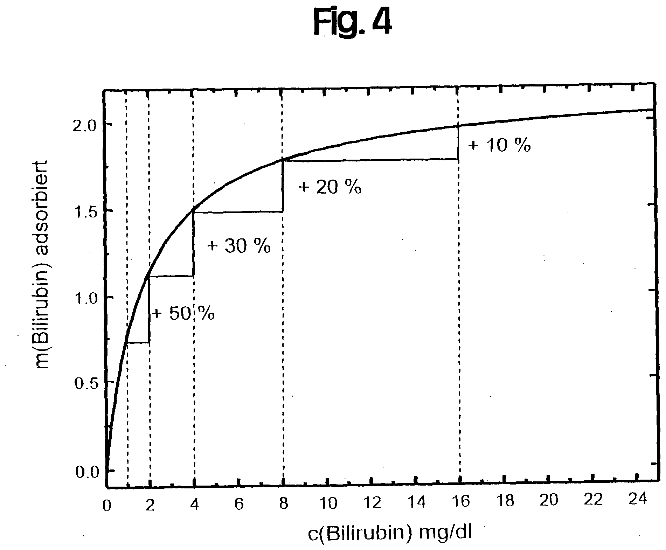

Figur 4 zeigt den theoretischen Effekt der ultrafiltrationsverstärkten Adsorption. In Figur 4 ist der Effekt dargestellt, den eine Konzentrationserhöhung um den Faktor 2 auf die adsorbierte Menge im Falle von Bilirubin und einem bekannten Adsorber bewirkt. Liegt die Konzentration im Plasma bei 8 mg/dl und wird das Bilirubin durch die Ultrafiltration auf 16 mg/dl aufkonzentriert, kann der Adsorber 10 % mehr Bilirubin adsorbieren.

Wie aus Figur 4 ersichtlich, erhöht sich die Steigerung der Adsorptionsleistung mit fallender Konzentration des Bilirubins. Liegt die Konzentration im Plasma durch Abnahme während der Behandlung z. B. bei 4 mg/dl, bewirkt eine Erhöhung um den Faktor 2 eine Steigerung der adsorbierten Menge um 20 %. Bei einer Konzentrationserhöhung von 1 mg/dl auf 2 mg/dl beträgt der Effekt sogar 50 %.

Erfordert die Therapie auch eine Eliminierung von kleinmolekularen Stoffen durch Dialyse, wie dies beispielsweise bei der Leberunterstützungstherapie der Fall ist, kann der Ultrafilter auch optional zur Dialyse mitgenutzt werden. Dies ist in den Figuren 2a, 2b und 3 angedeutet. In diesem Falle dient der Ultrafilter nicht nur der Aufkonzentrierung der im Ultrafilter nicht abgetrennten Komponenten, sondern auch der Abtrennung kleinmolekularer Stoffe aus dem Plasma. Der Ultrafilter wird somit auf einer Seite der Membran von Blutplasma und auf der anderen Seite von Dialysat durchströmt.

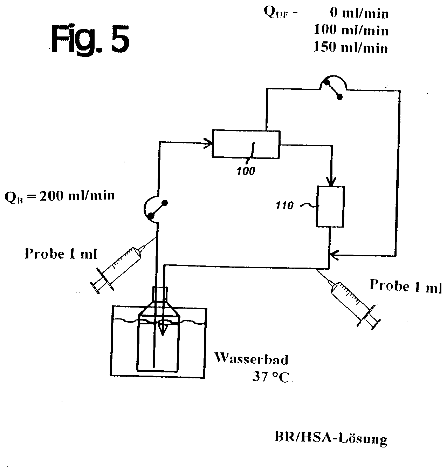

Figur 5 zeigt das Schema des Versuchsaufbaus zur Verifizierung des beschriebenen Effektes der Aufkonzentrierung der abzureichernden Komponente. Der Aufbau der Anordnung und die Durchführung des mit der Anordnung gemäß Figur 5 durchgeführten Versuches gestalten sich wie folgt:

Jeweils 1000 ml einer BR/HSA-Lösung (BR = Bilirubin; HSA = Humanes Serumalbumin) mit einer BR-Konzentration von 15 mg/dl und einer HSA-Konzentration von 30 g/l wurden drei Stunden über eine Adsorberkartusche mit einem Fluß von Q_{B} = 200 ml/min rezirkuliert. Mittels Filtration durch den Highflux-Dialysator 100 wurde die BR/HSA-Lösung vor der Adsorberkartusche 110 aufkonzentriert. Das Filtrat wurde dem Kreislauf nach der Adsorberkartusche 110 wieder zugeführt, wie dies in Figur 5 dargestellt ist.

Es wurden drei Versuche mit verschiedenen Filtrationsflussarten Q_{UF} durchgeführt. Der erste Versuch wurde mit Q_{UF} = 0 ml/min durchgeführt und diente als Kontrollversuch. Im zweiten Versuch wurde mit Q_{UF} = 100 ml/min die durch den Adsorber geleitete Lösung ca. um den Faktor 2 aufkonzentriert. Im dritten Versuch mit Q_{UF} = 150 ml/min etwa um den Faktor 4.

Zunächst wurde eine BR/HSA-Lösung so angesetzt, daß nach Zuführung des Füllvolumens der Adsorberkartusche 110 eine BR-Konzentration von 15 mg/dl und eine HSA-Konzentration von 30 g/l erreicht wurde. Dazu wurde zunächst 150 mg BR in 20 ml 0,1 N NaOH unter Rühren gelöst. Nach vollständigem Lösen des BR wurden 150 ml 20 % HSA-Lösung zugegeben und eine Stunde bei Raumtemperatur gerührt. Anschließend wurden 760 ml wäßrige 0,01 N Phosphatpufferlösung (pH 7,4) mit 110 mM NaCl zugegeben und im Wasserbad auf 37 ° C erwärmt.

Vor Beginn der Versuche wurde die Adsorberkartusche 110 mit 1000 ml isotonischer Kochsalzlösung gespült, wobei jeweils die letzten ca. 70 ml als Füllvolumen in der Adsorberkartusche 110 verblieben. Zu Beginn der Versuche wurde zunächst der Kreislauf bis zum Pegelstand des Füllvolumens der Adsorberkapsel sowie der Filtratkreislauf mit BR/HSA-Lösung bzw. Filtrat gefüllt. Danach wurden die Versuche gestartet, wobei mit der ersten sichtbaren Gelbfärbung der aus dem Adsorber 110 tretenden Lösung die ersten Proben entnommen wurden und die Zeitnahme auf 0 gesetzt wurde. Es wurden alle 15 Minuten jeweils 1 ml Proben vor und nach der Adsorberkartusche aus dem nicht aufkonzentrierten Bereich des Kreislaufs entnommen, wie dies in Figur 5 angedeutet ist. Im dritten Versuch mit der höchsten Ultrafiltrationsrate Q_{UF} = 150 ml/min wurden in den ersten 30 Minuten alle 5 Minuten Proben entnommen.

Die Zusammensetzung der 1000 ml BR/HSA-Lösung ist wie folgt:
- 150 mg Bilirubin
- 20 ml 0,1 N NaOH
- 150 ml 20 % HSA-Lösung
- 760 ml wäßrige 0,01 N Phosphatpufferlösung pH 7,4 mit 110 mM NaCl
- ca. 70 ml isotonische NaCI-Lösung (Füllvolumen Adsorber)

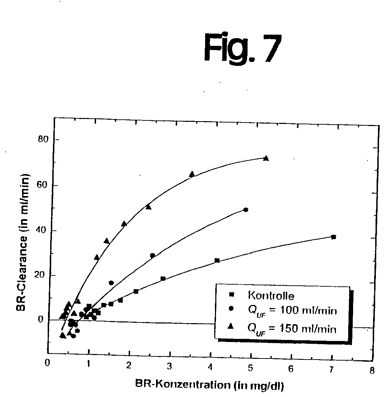

Die Zusammensetzung von 2000 ml 0,01 N Phosphatpuffer mit 110 mM NaCl ist wie folgt:
- 0,502 g NaH₂PO₄ H₂O
- 5,860 g Na₂HPO₄H₂O
- 12,860 g NaCl
- 2000 ml H₂O.

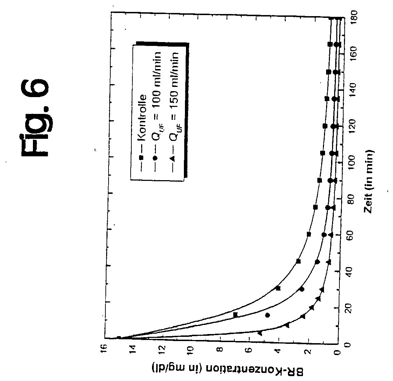

Figur 6 zeigt die Bilirubin-Konzentrationen der vor der Adsorberkartusche entnommenen Proben in Abhängigkeit von der Versuchsdauer. Zum Zeitpunkt t = 0 Minuten wurde der theoretische Ausgangskonzentrationswert von Bilirubin von 15 mg/dl eingesetzt. Es wurde nicht der gemessene Wert angesetzt, da zum Zeitpunkt t = 0 noch keine ausreichende Vermischung der BR/HSA-Lösung mit der in dem Absorber 110 befindlichen NaCI-Lösung vorlag.
Wie aus Figur 6 ersichtlich, ist mit zunehmender Aufkonzentrierung, d. h. mit steigender Ultrafiltrationsrate eine deutliche Beschleunigung der Abnahme der Bilirubin-Konzentration, d. h. eine Verbesserung der Reinigungsleistung durch Aufkonzentrierung vor der Adsorption zu erkennen. Figur 7 zeigt basierend auf den Ergebnissen gemäß Figur 6 die Clearance für Bilirubin in Abhängigkeit der BilirubinKonzentration der vor der Adsorberkartusche 110 entnommenen Proben. Die aus den jeweils zeitlich ersten Proben berechneten Clearance-Werte wurden nicht berücksichtigt, da hier noch keine ausreichende Vermischung der BR/HSA-Lösung mit der NaCI-Lösung des Füllvolumens des Absorbers 110 vorlag. Die Werte wurden mittels einer Exponentialfunktion 1. Ordnung gefittet.
Entsprechend der Versuchsergebnisse gemäß Figur 6 ist erkennbar, daß die Clearance des Adsorbers, d.h. dessen Reinigungsleistung mit steigender Ultrafiltrationsrate, d. h. mit steigender Konzentration der abzureichernden Spezies ansteigt.

Die erfindungsgemäße Vorrichtung ist allgemein für beliebige abreichernde Verfahren jenseits der Medizintechnik sowie auch im Bereich der Medizintechnik anwendbar. Mögliche Anwendungen sind Adsorptionsverfahren sowie auch andere abreichernde Verfahren, wie beispielsweise die Dialyse.

## Patentansprüche

1. Vorrichtung zur Abreicherung wenigstens einer Komponente eines fluiden Mediums, wobei die Vorrichtung eine Konzentrationserhöhungseinheit zur Erhöhung der Konzentration der abzureichernden Komponente des fluiden Mediums mittels Ultrafiltration umfaßt, wobei die Konzentrationserhöhungseinheit derart ausgeführt ist, dass die Konzentration der wenigstens einen abzureichernden Komponente in dem fluiden Medium vor deren Abreicherung erhöht wird, wobei die Vorrichtung eine der Konzentrationserhöhungseinheit nachgeschaltete und mit dieser in Fluidverbindung stehende, als Adsorber (60) ausgeführte Abreicherungseinheit zur anschließenden Verringerung der Konzentration der abzureichernden Komponente umfaßt, wobei die Vorrichtung ferner einen extrakorporalen Kreislauf (10) zur Führung von Blut oder einem oder mehreren Blutbestandteile umfasst, in dem die Konzentrationserhöhungseinheit sowie die Abreicherungseinheit angeordnet sind, **dadurch gekennzeichnet, dass** stromabwärts der Abreicherungseinheit eine Leitung in den extrakorporalen Kreislauf (10) mündet, die mit einer Quelle eines Substitutionsmediums in Fluidverbindung steht.

2. Vorrichtung nach Anspruch 1,
**dadurch gekennzeichnet, dass** die Konzentrationserhöhungseinheit als Membranvorrichtung, vorzugsweise als Ultrafilter (50) und als Trennvorrichtung, deren Trennwirkung auf der Einstellung eines thermodynamischen Phasengleichgewichtes beruht, ausgeführt ist.

3. Vorrichtung nach Anspruch 1 oder 2,
**dadurch gekennzeichnet, dass** die Abreicherungseinheit als Trennvorrichtung ausgeführt ist, deren Trennwirkung auf der Einstellung eines thermodynamischen Phasengleichgewichtes beruht, und als Membranvorrichtung , vorzugsweise als Dialysator, ausgeführt ist.

4. Vorrichtung nach Anspruch 1,
**dadurch gekennzeichnet, dass** von der Konzentrationserhöhungseinheit eine Leitung abzweigt, die stromabwärts der Abreicherungseinheit in den extrakorporalen Kreislauf (10) mündet.

5. Vorrichtung nach einem der Ansprüche 1 bis 4,
**dadurch gekennzeichnet, dass** der extrakorporale Kreislauf (10) einen Erst- und einen Zweitkreislauf (14) aufweist, wobei der Erstkreislauf (12) mit einem Patienten in Fluidverbindung steht oder verbindbar ist und wobei der Zweitkreislauf (14) mit dem Erstkreislauf (12) mittels eines Plasmafilters (20) in Verbindung steht und die Konzentrationserhöhungseinheit sowie die Abreicherungseinheit umfasst.

6. Vorrichtung nach einem der vorhergehenden Ansprüche,
**dadurch gekennzeichnet, dass** an die Konzentrationserhöhungseinheit ein Dialysat-Kreislauf angeschlossen ist.

## Claims

1. An apparatus for the depletion of at least one component of a fluid medium, wherein the apparatus comprises a concentration increasing unit for increasing the concentration of the component of the fluid medium to be depleted by means of ultrafiltration, with the concentration increasing unit being configured such that the concentration of the component in the fluid medium to be depleted is increased before being depleted, wherein the apparatus comprises a depletion unit, configured as adsorber (60), disposed downstream of the concentration increasing unit and in fluid communication therewith for the subsequent reduction in the concentration of the component to be depleted, wherein the apparatus further comprises an extracorporeal circuit (10) for the conducting of blood or one or more blood components in which the concentration increasing unit and the depletion unit are arranged,
**characterized in that** a line opens into the extracorporeal circuit (10) downstream of the depletion unit, which is in fluid communication with a source of a substitution medium.

2. An apparatus in accordance with claim 1,
**characterized in that** the concentration increasing unit is configured as a membrane apparatus, preferably as an ultrafilter (50) and as a separating apparatus whose separation effect is based on the setting of a thermodynamic phase equilibrium.

3. An apparatus in accordance with claim 1 or 2,
**characterized in that** the depletion unit is configured as a separating apparatus, whose separation effect is based on the setting of a thermodynamic phase equilibrium, and as a membrane apparatus, preferably as a dialyzer.

4. An apparatus in accordance with claim 1,
**characterized in that** a line that opens into the extracorporeal circuit (10) downstream of the depletion unit branches from the concentration increasing unit.

5. An apparatus in accordance with any one of claims 1 to 4,
**characterized in that** the extracorporeal circuit (10) has a first and a second circuit (14), with the first circuit (12) being in fluid communication with or being connectable to a patient and with the second circuit (14) being in communication with the first circuit (12) by means of a plasma filter (20) and including the concentration increasing unit and the depletion unit.

6. An apparatus in accordance with any one of the preceding claims,
**characterized in that** a dialysate circuit is connected to the concentration increasing unit.

## Revendications

1. Dispositif servant à appauvrir au moins un composant d'un milieu fluide, dans lequel le dispositif comprend une unité d'augmentation de concentration servant à augmenter la concentration du composant à appauvrir du milieu fluide au moyen d'une ultrafiltration, dans lequel l'unité d'augmentation de concentration est réalisée de telle manière que la concentration de l'au moins un composant à appauvrir dans le milieu fluide est augmentée avant son appauvrissement, dans lequel le dispositif comprend une unité d'appauvrissement installée en aval de l'unité d'augmentation de concentration et en communication fluidique avec celle-ci, réalisée comme adsorbeur (60), servant à réduire par la suite la concentration du composant à appauvrir, dans lequel le dispositif comprend en outre un circuit extracorporel (10) servant à guider du sang ou un ou plusieurs composants sanguins, dans lequel l'unité d'augmentation de concentration ainsi que l'unité d'appauvrissement sont disposées, **caractérisé en ce que** débouche, en aval de l'unité d'appauvrissement, dans le circuit extracorporel (10), un conduit, qui est en communication fluidique avec une source d'un milieu de substitution.

2. Dispositif selon la revendication 1,
**caractérisé en ce que** l'unité d'augmentation de concentration est réalisée sous la forme d'un dispositif à membrane, de préférence sous la forme d'un ultrafiltre (50) et sous la forme d'un dispositif de séparation, dont l'action de séparation repose sur le réglage d'un équilibre de phases thermodynamiques.

3. Dispositif selon la revendication 1 ou 2,
**caractérisé en ce que** l'unité d'appauvrissement est réalisée sous la forme d'un dispositif de séparation, dont l'action de séparation repose sur le réglage d'un équilibre de phases thermodynamiques, et est réalisée sous la forme d'un dispositif à membrane, de préférence sous la forme d'un dialyseur.

4. Dispositif selon la revendication 1,
**caractérisé en ce que** dévie de l'unité d'augmentation de concentration un conduit, qui débouche dans le circuit extracorporel (10) en aval de l'unité d'appauvrissement.

5. Dispositif selon l'une quelconque des revendications 1 à 4,
**caractérisé en ce que** le circuit extracorporel (10) présente un premier circuit et un deuxième circuit (14), dans lequel le premier circuit (12) est en communication fluidique avec un patient ou peut être relié à ce dernier et dans lequel le deuxième circuit (14) est en communication avec le premier circuit (12) au moyen d'un filtre à plasma (20) et comprend l'unité d'augmentation de concentration ainsi que l'unité d'appauvrissement.

6. Dispositif selon l'une quelconque des revendications précédentes,
**caractérisé en ce qu'**un circuit à dialysat est raccordé à l'unité d'augmentation de concentration.
